# EUROPEAN PATENT APPLICATION

(11) **EP 1 800 676 A1**
(43) Date of publication of application: **27.06.2007**
(21) Application number: 06126873.6
(22) Date of filing: 21.12.2006
(51) Int. Cl.: A61K 31/34, A61K 31/38, A61K 31/416, A61K 31/519, A61P 31/00, A61P 35/00

(54) **Cancer chemotherapy**

(30) Priority: 23.12.2005 US 753385 P
(71) Applicant: Yung Shin Pharmaceutical Ind. Co., Ltd., Tachia T'ai chung (TW)
(72) Inventor: Kuo, Sheng-Chu, Taichung (TW); Teng, Che-Ming, 106 Taipei (TW); Lee, Fang-Yu, Taichung (TW); Pan, Shiow-Lin, Da-an District Taipei (TW); Guh, Jih-Hwa, Sin-yi District 110 Taipei (TW)
(74) Representative: Weihs, Bruno Konrad

(57) **Abstract**

A method for treating cancer, which includes administrating to a subject in need thereof an effective amount of a chemotherapeutic agent and an effective amount of a compound of the formula: 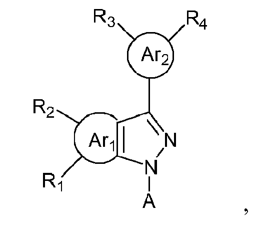 in which, A is H or 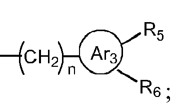 each of Ar₁, Ar₂, and Ar₃, independently, is phenyl, thienyl, furyl, pyrrolyl, pyridinyl, or pyrimidinyl; each of R₁, R₂, R₃, R₄, R₅, and R₆, independently, is R, nitro, halogen, C(O)OR, C(O)SR, C(O)NRR', (CH₂)ₘOR, (CH₂)ₘSR, (CH₂)ₘNRR', (CH₂)ₘCN, (CH₂)ₘC(O)OR, (CH₂)ₘCHO, (CH₂)ₘCH=NOR, (CH₂)ₘC(O)N(OR)R', N(OR)R', or R₁ and R₂ together, R₃ and R₄ together, or R₅ and R₆ together are O(CH₂)ₘO, in which each of R and R', independently, is H or C₁~C₆ alkyl; and m is 0, 1, 2, 3, 4, 5, or 6, and n is 0, 1, 2, or 3.

## Description

### Field of the Invention

The present invention relates to methods and compositions that can improve the efficacy of cancer chemotherapy.

### Background of the Invention

Cancers, a leading fatal disease, feature an abnormal mass of malignant tissue resulting from excessive cell division. Cancer cells proliferate in defiance of normal restraints on cell growth, and invade and colonize territories normally reserved for other cells. Cancers may be treated by surgery, radiotherapy, chemotherapy, hormonal therapy, etc.

Chemotherapy is the use of anti-cancer drugs (or cytotoxic drugs) to inhibit or destroy cancer cells. It inhibits cancer cell growth by targeting specific parts of the cell growth cycle. Currently, there are more than 50 chemotherapeutic drugs approved for clinical use, and hundreds more are being investigated. Chemotherapy drugs can stop cancer cells from further proliferating by various mechanisms. Most chemotherapeutic agents take advantage of the fact that cancerous cells are more active in cell growth and division. Therefore, drugs that interfere with cell growth cycles or cell division processes will selectively inhibit cancer cell growth.

For example, Doxorubicin (Adriamycin, Rubex, or Doxil) is an anthracycline antibiotic that exerts its effects on cancer cells via intercalation and enzyme inhibition. As an intercalator, it inserts into DNA and prevents DNA from replicating or being transcribed. As an enzyme inhibitor, it inhibits topoisomerase type II, leading to DNA breaks. Because actively dividing cancer cells need to have DNA synthesis and replication, Doxorubicin will impact the actively dividing cells (i.e., cancer cells) more than it does to normal cells. Because the action mechanisms of Doxorubicin is general, this drug is useful in the treatment of a wide range of cancers.

Paclitaxel (taxol) or its analogs (such as Docetaxel (Taxotere)) promotes the polymerization of tubulin, thereby causing cell death by disrupting the normal microtubule dynamics required for cell division and vital interphase processes.

Vitamin A metabolites, retinoic acids (RAs), have been known to have profound effects on development, cellular proliferation and differentiation, and tumor growth and invasion. The wide range effects of RAs on cellular proliferation and migration suggest that they are useful chemotherapeutic agents for many types of cancer. For example, all trans retinoic acid (ATRA) has been successfully used as "differentiation therapy" for various leukemia, such as acute promyelocytic leukemia (APL). ATRA activates the retinoid receptor (RAR) and causes the promyelocytes to differentiate (to mature), preventing such cells from proliferating.

Although chemotherapy has greatly improved in efficacy and reduced side effects over the years, the overall curable rate in cancer chemotherapy is still low and far from satisfaction. Thus, there remains a need for more effective chemotherapy.

### SUMMARY

This invention is based on a surprising discovery that certain fused pyrazolyl compounds can be used with chemotherapeutic agents to produce synergistic effects. Such synergistic effects are observed with chemotherapeutic agents that work by different mechanisms. Thus, the fused pyrazolyl compounds of the invention may be generally used with other chemotherapeutic agents, such as paclitaxel, doxorubicin, or retinoic acid, to improve the efficacies and/or to minimize the size effects of the chemotherapeutic agents.

In one aspect, this invention features a method for treating cancer. The method includes administrating to a subject in need thereof an effective amount of a chemotherapeutic agent (e.g., paclitaxel, doxorubicin, or retinoic acid) and an effective amount of a compound of the formula:

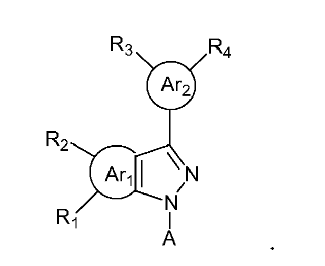

In this formula, A is H or 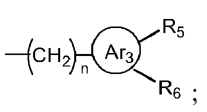 each of Ar₁, Ar₂, and Ar₃, independently, is phenyl, thienyl, furyl, pyrrolyl, pyridinyl, or pyrimidinyl; each of R₁, R₂, R₃, R₄, R₅, and R₆, independently, is R, nitro, halogen, C(O)OR, C(O)SR, C(O)NRR', (CH₂)ₘOR, (CH₂)ₘSR, (CH₂)ₘNRR', (CH₂)ₘCN, (CH₂)ₘC(O)OR, (CH₂)ₘCHO, (CH₂)ₘCH=NOR, (CH₂)ₘC(O)N(OR)R', N(OR)R', or R₁ and R₂ together, R₃ and R₄ together, or R₅ and R₆ together are O(CH₂)ₘO, in which each of R and R', independently, is H or C₁~C₆ alkyl; and m is 0, 1, 2, 3, 4, 5, or 6, and n is 0, 1, 2, or 3. (CH₂)ₘ can be branched or linear. Note that the left atom shown in any substituted group described above is closest to the fused pyrazolyl ring. Also note that when there are one or more R or (CH₂)ₘ moieties in a fused pyrazolyl compound, the R or the (CH₂)ₘ moieties can be the same or different.

In some of the above-described compounds, Ar₁ can be phenyl, Ar₂ can be furyl (e.g., 5'-furyl), and Ar₃ can be phenyl.

A subset of the above-described compounds is those in which A is 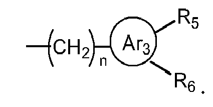 In some particular embodiments, Ar₁ is phenyl, Ar₂ is 5'-furyl, Ar₃ is phenyl, and n is 1. Further, each of R₁, R₂, R₅, and R₆ is H, n is 1, and one of R₃ and R₄ is H, and the other is CH₂OH substituted at position 2 of furyl. An example of this subset is 1-benzyl-3-(5'-hydroxymethyl-2'-furyl)indazole (Compound 1).

Another subset of the above described compounds are those in which A is H. In some particular embodiments, Ar₁ is phenyl and Ar₂ is furyl. Further, each of R₁ and R₂ is H, and one of R₃ and R₄ is H, and the other is CH₂OH substituted at position 2 of furyl.

The term "Ar," as used herein, refers to both aryl and heteroaryl groups. Aryl, e.g., phenyl, is a hydrocarbon ring system having at least one aromatic ring. Heteroaryl is a hydrocarbon ring system having at least one aromatic ring which contains at least one heteroatom such as O, N, or S. Examples of heteroaryl include, but are not limited to, thienyl, furyl, pyrrolyl, pyridinyl, and pyrimidinyl. An "Ar" may contain one, two, three, or more substituents on its ring. In addition to those assigned to R₁, R₂, R₃, R₄, R₅, and R₆ (see above), the substituents can also be nitro, C₂~C₆ alkenyl, C₂~C₆ alkynyl, aryl, heteroaryl, cyclyl, or heterocyclyl. Alkyl, alkenyl, alkynyl, alkoxy, aryl, heteroaryl, cyclyl, and heterocyclyl, as used herein, are optionally substituted with C₁~C₆ alkyl, halogen, amino, hydroxyl, mercapto, cyano, or nitro. Note that the term "alkyl" refers to both linear alkyl and branched alkyl.

The fused pyrazolyl compounds described above include the compounds themselves, as well as their salts and their prodrugs, if applicable. Such salts, for example, can be formed by interaction between a negatively charged substituent (e.g., carboxylate) on a fused pyrazolyl compound and a cation. Suitable cations include, but are not limited to, sodium ion, potassium ion, magnesium ion, calcium ion, and an ammonium cation such as teteramethylammonium ion. Likewise, a positively charged substituent (e.g., amino) can form a salt with a negatively charged counterion. Suitable counterions include, but are not limited to, chloride, bromide, iodide, sulfate, nitrate, phosphate, or acetate. Examples of prodrugs include esters and other pharmaceutically acceptable derivatives, which, upon administration to a subject, are capable of providing the fused pyrazolyl compounds described above.

A chemotherapeutic agent for use with a fused pyrazolyl compound in accordance with embodiments of the invention may function by various mechanisms. For example, it may function by DNA intercalation, by modulating microtubule dynamics, by inducing cell differentiation, by inducing apoptosis, or any mechanism that modulate cell cycles.

The "cancer" as used in this description includes cellular tumors/cancers, lymphoma, and leukemia. The term is meant to include all types of cancerous growths, metastatic tissues or malignantly transformed cells, tissues, or organs, irrespective of histopathologic type, or stage of invasiveness. Examples of cancers include, but are not limited to, carcinoma and sarcoma, such as leukemia, sarcomas, osteosarcoma, lymphomas, melanoma, ovarian cancer, skin cancer, testicular cancer, gastric cancer, pancreatic cancer, renal cancer, breast cancer, prostate cancer, colorectal cancer, cancer of head and neck, brain cancer, esophageal cancer, bladder cancer, adrenal cortical cancer, lung cancer, bronchus cancer, endometrial cancer, nasopharyngeal cancer, cervical cancer, hepatic cancer, or cancer of unknown primary site.

Also within the scope of this invention is a composition containing one or more of the fused pyrazolyl compounds described above and a chemotherapeutic agent for use in treating cancer, and the use of this composition for the manufacture of a medicament for the treatment of cancer.

Other features, objects, and advantages of the invention will be apparent from the description, and from the claims.

### BRIEF DESCRIPTION OF THE DRAWINGS

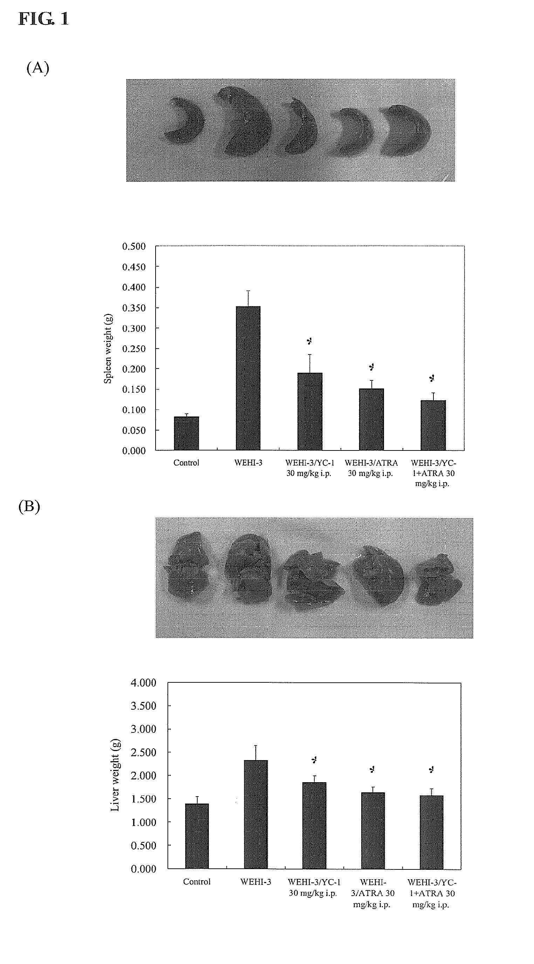

FIG. 1 shows changes of spleen (A) and liver (B) weight of BALB/c and WEHI-3/BALB/c mice with or without compound 1 (YC-1), ATRA, compound 1 combined with ATRA administrations (n=8). The results were expressed as mean±S.D. and the difference between the groups was tested by one way ANOVA. Significant differences between values for WEHI-3B/BALB/c mice and various treatment are shown * *p* < 0.05.

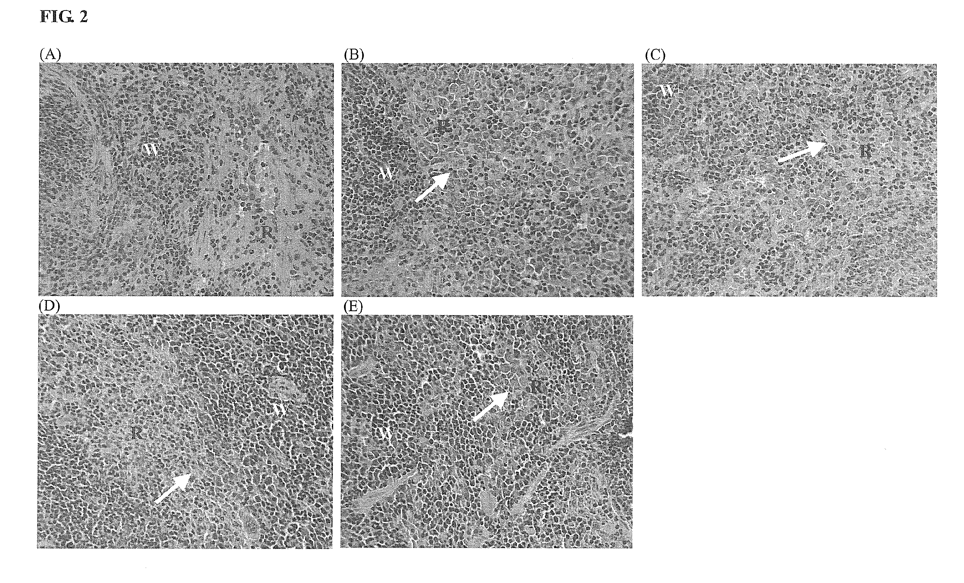

FIG. 2 shows Hematoxylin and eosin staining of paraffin sections from spleen of control mice (A), WEHI-3/BALB/c leukemia mice (B), compound 1 treated leukemia mice (C), ATRA treated leukemia mice (D) and compound 1 combined ATRA treated leukemia mice (E). After 14 days of treatment, mice were sacrificed. Spleen were fixed in 4% formaldehyde and embedded in paraffin. Spleen sections were stained with hematoxylin and eosin. (R: red pulp; W: while pulp;)↓: infiltrated immature myeloblastic cells)

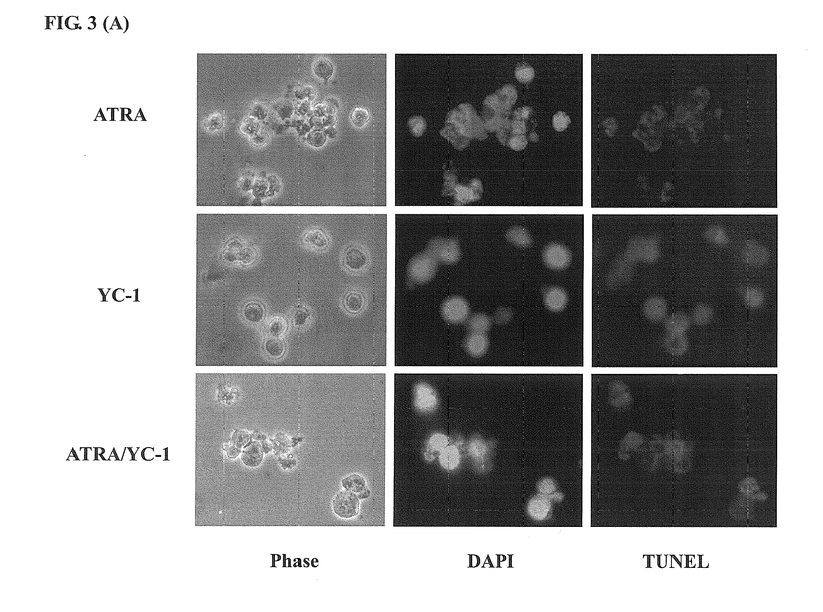

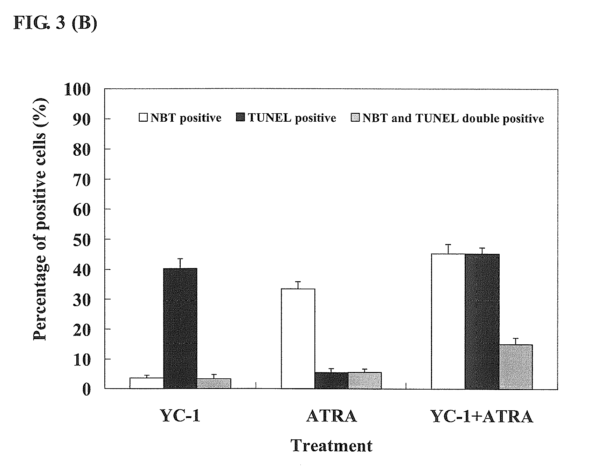

FIG. 3 shows effects of compound 1, ATRA and compound 1 combined with ATRA on differentiation and apoptosis of WEHI-3 cells. (A) Nuclear morphological changes induced by TUNEL/DAPI staining (B) NBT reduction and TUNEL staining. compound 1, ATRA, compound 1 combined with ATRA-treated WEHI-3 cells cultured for 24 h before all treatment were stained with TUNEL/DAPI (x400) followed by microscopic analysis. The results were expressed as mean±S.D.

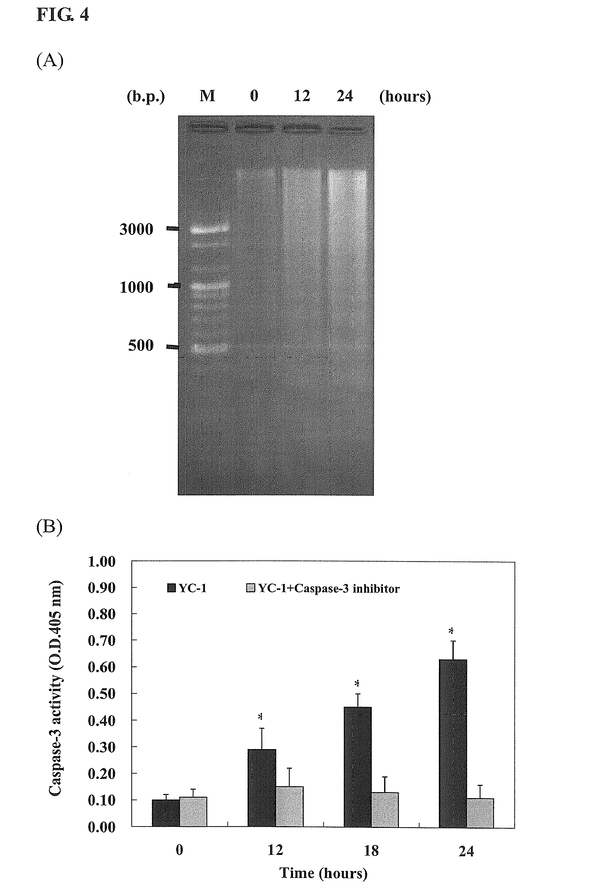

FIG. 4 shows effects of compound 1 induced apoptosis in WEHI-3 cells by (A) DNA fragmentation and (B) caspase-3 activity. For DNA fragmentation, DNA extracts were electrophoresed in 1.5 % agarose gel. Effects of caspase-3 inhibitor on YC-induced caspase-3 activation. Cells were pretreated with caspase-3 inhibitor, Z-DEVE-FMK, for 1 h and then stimulated with 5 µM of compound 1. The results were expressed as mean± S.D. and the difference between the groups was tested by one way ANOVA. Significant differences between values for 0 hour-treatment and various hours treatment are shown * p < 0.001.

### DETAILED DESCRIPTION

This invention relates to a method for treating cancer (e.g., renal cancer, lung cancer, kidney cancer, or leukemia) by administering to a subject, who needs the treatment, an effective amount of one or more fused pyrazolyl compounds and an effective amount of a chemotherapeutic agent. The term "treating" refers to the application or administration of a fused pyrazolyl compound (or a composition comprising a fused pyrazolyl compound) and a chemotherapeutic agent to a subject, who has cancer, a symptom of cancer, or a predisposition toward cancer, with the purpose to cure, heal, alleviate, relieve, alter, remedy, ameliorate, improve, or affect the cancer, the symptoms of the cancer, or the predisposition toward the cancer. "An effective amount" refers to the amount of an active agent which, upon administration to a subject who needs the treatment, is required to confer therapeutic effect on the subject. Effective doses may vary, as recognized by those skilled in the art, depending on the route of administration, excipient usage, and the possibility of co-usage with other agents for treating an angiogenesis-related disorder.

A fused pyrazolyl compound used to practice a method of this invention can be prepared by procedures well known to a skilled person in the art. For example, U.S. Patent No. 5,574,168, which is assigned to the assignee of the present invention and is incorporated by reference in its entirety, describes procedures including the following synthetic steps: An aryl aryl ketone is first prepared by coupling an arylcarbonyl chloride with another aryl compound. Either aryl compound is optionally mono- or multi-substituted. The ketone then reacts with an arylalkylhydrazine, the aryl group of which is also optionally mono- or multi-substituted, to form a hydrazone containing three aryl groups. The hydrazone group is transformed into a fused pyrazolyl core via an alkylene linker, another aryl group is fused at 4-C and 5-C of the pyrazolyl core, and the third aryl group is directly connected to 3-C of the pyrazolyl core. Derivatives of the fused pyrazolyl compound may be obtained by modifying the substituents on any of the aryl groups.

The chemicals used in the above synthetic route may include, for example, solvents, reagents, catalysts, protecting group and deprotecting group reagents. The synthetic route may also additionally include steps, either before or after the steps described specifically herein, to add or remove suitable protecting groups in order to ultimately allow synthesis of the fused pyrazolyl compound. In addition, various synthetic steps may be performed in an alternate sequence or order to give the desired compounds. Synthetic chemistry transformations and protecting group methodologies (protection and deprotection) useful in synthesizing applicable fused pyrazolyl compounds are known in the art and include, for example, those described in R. Larock, Comprehensive Organic Transformations, VCH Publishers (1989); T.W. Greene and P.G.M. Wuts, Protective Groups in Organic Synthesis, 2d. Ed., John Wiley and Sons (1991); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) and subsequent editions thereof.

A fused pyrazolyl compound thus synthesized can be further purified by a method such as column chromatography, high-performance liquid chromatography, or recrystallization.

Fused pyrazolyl compounds in accordance with embodiments of the invention have been found to have anti-angiogenic effects and anti-cancer effects, as disclosed in U.S. Patent Application Nos. 2003/0186996, 2005/0107406, and 2005/0209252, which are all by Teng et al. and are assigned to the assignee of the present invention. These applications are incorporated by reference in their entireties.

For example, 1-benzyl-3-(5'-hydroxymethyl-2'-furyl)indazole) (compound 1 to be described in detail later) has been found to be a novel antiplatelet agent. Mechanism studies revealed that the antiplatelet activity of compound 1 was associated with NO-independent activation of soluble guanylyl cyclase (sGC). Recently, compound 1 was shown to have anticancer activity; it blocks tumor angiogenesis via suppressing hypoxia-inducible factor-α (HIF-1) activity and arresting cell cycles at the Go/G1 stage in human hepatocellular carcinoma HA22T cells by increasing the expression of cyclin-dependent kinase (CDK)-inhibitory protein p21^{CIP1/WAP1} and p27^{KIP1}. Compound 1 also inhibits hypoxia induction of erythropoietin and vascular endothelial growth factor in Hep3B cells.

Although compound 1 was previously found to have anti-cancer effects towards certain cancers, the inventors of the present invention have unexpectedly found that pyrazolyl compounds of the present invention can produce synergistic effects with other chemotherapeutic agents. A chemotherapeutic agent used to practice the method of this invention is commercially available or can be synthesized by the procedures well known in the art.

A chemotherapeutic agent for use with a fused pyrazolyl compound in accordance with embodiments of the invention may function by various mechanisms. For example, it may function by DNA intercalation, by modulating microtubule dynamics, by inducing cell differentiation, by inducing apoptosis, or any mechanism that modulate cell cycles.

A DNA-intercalating or DNA-binding chemotherapeutic agent can bind or insert itself into the double stranded DNA. As a result, DNA dependent RNA transcription is inhibited. In addition, DNA replication is also hampered. Some of these DNA binding agents can also inhibit topoisomerase I, which is essential during DNA replication. Therefore, these DNA binding agents can lead to DNA single or double strand breaks. Some single strand breaks may be repaired by the DNA repair system in cells before cells enter mitosis. However, part of the single strand breaks that are not repaired and DNA double strand breaks may cause cell chromosome aberrations and even cell death. Examples of DNA-binding or intercalating chemotherapeutic agents may include, but are not limited to doxorubicin, daunorubicin, dactinomycin, cyclophosphamide, and mitomycin-C.

Microtubule-modulating agents interfere with the assembly and disassembly of microtubule cytoskeletons. Microtubules are formed by polymerization of tubulins. The polymerization and depolymerization of microtubules are controlled processes because microtubule skeletons play important roles in various aspects of cell functions. Agents that can interfere with the polymerization or depolymerization of microtubules can be used to modulate cellular activities. Colchicine, colcemid, and nocadazol inhibit polymerization by binding to tubulin and preventing its addition to the plus ends of a growing microtubule. Vinblastine and vincristine aggregate tubulin and lead to microtubule depolymerization, resulting in blocking mitosis by arresting cells in the metaphase. Taxol (paclitaxel) or epothilone stabilizes microtubules by binding to the microtubules.

Cancer can result from deranged cell division or inability of cells to differentiate into mature cell types. Therefore, differentiation inducing agents may be used to induce the cancer cells to differentiate. Examples of differentiation therapeutic agents include retinoic acid metabolites and derivatives. For example, all trans retinoic acid (ATRA) has been successfully used as "differentiation therapy" for various leukemia, such as acute promyelocytic leukemia (APL). ATRA activates the retinoid receptor (RAR) and causes the promyelocytes to differentiate (to mature), preventing such cells from proliferating.

Examples of other chemotherapeutic agents and their mechanisms include, but are not limited to, cisplatin (cis-diamminedichloroplatinum(II)), which induces its cytotoxic effects by binding to nuclear DNA to interference with normal transcription and/or DNA replication; bleomycin, which cause DNA cleavage by radical formation; topotecan, irinotecan, or camptothecin, which inhibits topoisomerase I; podophyllotoxin, which inhibits microtubule assembly in the mitotic apparatus; plicamycin, which binds to DNA and inhibits DNA, RNA, and protein synthesis in a manner similar to dactinomycin; or 5-fluorouracil, which inhibits DNA synthesis by inhibiting the normal production of thymidine.

The above described are commonly used chemotherapeutic agents and their mechanisms of actions. One of ordinary skill in the art would appreciate that these are illustrate examples and that embodiments of the invention are not limited to these examples. Instead, in accordance with embodiments of the invention, a fused pyrazolyl compound may be used with any known chemotherapeutic agents.

To practice the method of the present invention, a fused pyrazolyl compound and a chemotherapeutic agent can be administered at the same time or at different times. For example, one can administer to a cancer patient a fused pyrazolyl compound (or a composition containing a fused pyrazolyl compound) first, and then administer to the patient a pharmaceutically acceptable carrier and a composition containing a chemotherapeutic agent and a pharmaceutically acceptable carrier.

In another example, the active agents are administered at the same time, e.g., using a composition containing a fused pyrazolyl compound, a chemotherapeutic agent, and a pharmaceutically acceptable carrier. Any of the above-described composition can be administered orally, parenterally, by inhalation spray, or via an implanted reservoir. The term "parenteral" as used herein includes subcutaneous, intracutaneous, intravenous, intramuscular, intraarticular, intraarterial, intrasynovial, intrastemal, intrathecal, intralesional and intracranial injection or infusion techniques.

A composition for oral administration can be any orally acceptable dosage form including, but not limited to, tablets, capsules, emulsions and aqueous suspensions, dispersions and solutions. Commonly used carriers for tablets include lactose and corn starch. Lubricating agents, such as magnesium stearate, are also typically added to tablets. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added.

A sterile injectable composition (e.g., aqueous or oleaginous suspension) can be formulated according to techniques known in the art using suitable dispersing or wetting agents (such as, for example, Tween 80) and suspending agents. The sterile injectable preparation can also be a sterile injectable solution or suspension in a non-toxic parenterally acceptable diluent or solvent, for example, as a solution in 1,3-butanediol. Among the acceptable vehicles and solvents that can be employed are mannitol, water, Ringer's solution and isotonic sodium chloride solution. In addition, sterile, fixed oils are conventionally employed as a solvent or suspending medium (e.g., synthetic mono- or diglycerides). Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions can also contain a long-chain alcohol diluent or dispersant, or carboxymethyl cellulose or similar dispersing agents.

An inhalation composition can be prepared according to techniques well-known in the art of pharmaceutical formulation and can be prepared as solutions in saline, employing benzyl alcohol or other suitable preservatives, absorption promoters to enhance bioavailability, fluorocarbons, and/or other solubilizing or dispersing agents known in the art.

A carrier in a pharmaceutical composition must be "acceptable" in the sense of being compatible with the active ingredient of the formulation (and preferably, capable of stabilizing it) and not deleterious to the subject to be treated. For example, solubilizing agents, such as cyclodextrins (which form specific, more soluble complexes with fused pyrazolyl compounds), can be utilized as pharmaceutical excipients for delivery of fused pyrazolyl compounds. Examples of other carriers include colloidal silicon dioxide, magnesium stearate, cellulose, sodium lauryl sulfate, and D&C Yellow # 10.

The above-described method may further include radiation treatment. Radiation can be applied to the cancer site in a patient, before, during, or after the patient is administered with a desired dose of a fused pyrazolyl compound and a chemotherapeutic agent. The radiation may be ionizing radiation and non-ionizing radiation. Ionizing radiation has sufficient energy to interact with an atom and remove electrons from their orbits, causing the atom to become charged or "ionized." It includes radiation with gamma ray, X-ray, neutrons, electrons, alpha particles, and beta particles. Non-ionizing radiation is electromagnetic radiation that does not have sufficient energy to remove electrons from their orbits. It includes radiation with ultraviolet rays, visible light, infrared light, microwave, and radio waves. The radiation dose and time should be adequate to confer the therapeutic effect to the treated patient. It may vary, as recognized by those skilled in the art, depending on the type and intensity of the radiation, the type and location of the cancer to be treated, and the physical condition of the patient.

Suitable *in vitro* assays can be used to preliminarily evaluate the efficacy of a combination of one or more of the above-described compound and a chemotherapeutic agent in inhibiting growth of certain cancer cell lines. The combination can further be examined for its efficacy in treating cancer by *in vivo* assays. For example, the combination can be administered to an animal (e.g., a mouse model) having cancer and its therapeutic effect is then accessed. Based on the results, an appropriate dosage range and administration route can also be determined.

Without further elaboration, it is believed that the above description has adequately enabled the present invention. The following specific embodiments are, therefore, to be construed as merely illustrative, and not limitative of the remainder of the disclosure in any way whatsoever. All of the publications, including patents, cited herein are hereby incorporated by reference in their entirety.

### Synthesis of 1-benzyl-3-(5'-hydroxymethyl-2'-furyl)indazole (Compound 1)

Calcium borohydride was first prepared by stirring anhydrous calcium chloride (88.8 mg, 0.8 mmole) with sodium borohydride (60 mg, 1.6 mmole) in anhydrous THF (20 mL) for 4 hrs. Then a 30 mL THF solution containing 88.0 mg 1-benzyl-3-(5'-methoxycarbonyl-2'-furyl)indazole (0.27 mmole) was added dropwise to the calcium borohydride solution at 30±2 °C. The mixture was heated under reflux for 6 hrs, cooled, quenched into crushed ice, placed at a reduced pressure to remove THF, and filtered to obtain a solid product. The solid was extracted with dichloromethane. The extract was concentrated to 50 mL and a solid precipitated after petroleum ether was added. The precipitate was collected and purified by column chromatography (silica gel-benzene) to obtain 70.0 mg 1-benzyl-3-(5'-hydroxymethyl-2'-furyl)indazole at a yield of 87%.
mp: 108-109°C.
MS (%), m/z: 304 (M⁺).
IR (KBr) νₘₐₓ: 3350 cm⁻¹ (-OH).
¹H-NMR (DMSO-d₆, 200 MHz) δ: 4.51 (2H, d, J=5.5 Hz, -CH₂O-) 5.31 (1H, t, J=5.5 Hz, -OH), 5.70 (2H, s, =NCH₂-), 6.48 (1H, d, J=3.4 Hz, H-4'), 6.97 (1H, d, J=3.4 Hz, H-3'), 7.21-7.31 (6H, m, H-5, phenyl), 7.45 (1H, t, J=8.2 Hz, H-6), 7.75 (1H, dd, J=8.2, 1.8 Hz, H-7), 8.12 (1H. dd, J=8.2. 1.0 Hz. C4-H).

### Inhibitory effect on cancer cell growth:

Athymic nude mice (BALB/c nu/nu female, 6-8 week old) were housed at 22°C on a 12 h light/dark cycle. The mice were subcutaneously injected with A498 renal cancer cells (10⁷ cell/mouse). After inoculation for 15 days, tumors were grown to a size of 100 to 150 mm³. The mice were then randomly divided into six grounds (5 mice in each group). The mice of Groups 1 (the control group) and 2 were orally treated with 0.5% carboxymethyl cellulose (CMC) and Compound 1 in 0.5% CMC (10 mg/kg/day), respectively. The mice of Groups 3 and 4 were intraperitoneally injected with paclitaxel and doxorubicin (20 mg/kg/week), respectively. (paclitaxel and doxorubicin are commercial products). The mice of Group 5 were first orally treated with Compound 1 in 0.5% CMC (10 mg/kg/day) and then immediately injected intraperitoneally with paclitaxel (20 mg/kg/week). The mice of Group 6 were first orally treated with Compound 1 in 0.5% CMC (10 mg/kg/day) and then immediately injected intraperitoneally with doxorubicin (20 mg/kg/week). The tumor size in each mouse was measured every 3 to 4 days. The mice were euthanatized with intraperitoneal administration of pentobarbital when the tumor size of the control group reached 1000-1500 mm³. The tumors were carefully removed and weighed.

The results show that the mice treated with Compound 1, paclitaxel, or doxorubicin alone had smaller tumors than those in the control group. They also show that the mice treated with Compound 1 and paclitaxel combined or Compound 1 and doxorubicin combined had unexpectedly smaller tumor than those treated with Compound 1, paclitaxel, or doxorubicin alone.

### Synergistic Effects of Compound 1 and All Trans Retinoic Acid (ATRA) in the Treatment of Leukemia

Male BALB/c mice of 22-28 g in weight at the age of 8 weeks were obtained from Laboratory Animal Center, National Taiwan University College of Medicine (Taipei, Taiwan). Murine myelomonocytic leukemia cell line WEHI-3 was obtained from the Food Industry Research and Development Institute (Hsinchu, Taiwan). Murine WEHI-3 leukemia cell line was first established in 1969 and showed characteristics of myelomonocytic leukemia. This cell line can induce leukemia in syngenic BALB/c mice for evaluating anti-leukemia effects of drugs. (*see* He Q, Na X, "The effects and mechanisms of a novel 2-aminosteroid on murine WEHI-3B leukemia cells in vitro and in vivo," Leuk. Res. 25(6):455-61, (2001)). The cells were placed into 75-cm² tissue culture flasks and grown at 37 °C under a humidified 5% CO₂ atmosphere in RPMI 1640 medium supplemented with 10% fetal bovine serum, 1% penicillin-streptomycin (10,000 U/ml penicillin and 10 mg/ml streptomycin) and 1% glutamine.

To test the effects of compound 1, ATRA and compound 1 combined ATRA the survival of BALB/c mice bearing WEHI-3 leukemia cells (WEHI-3/BALB/c mice), compound 1, ATRA, and compound 1 combined with ATRA were administered i.p. 30 mg/kg/2 days for 14 days to BALB/c mice that had been inoculated with 1 × 10⁵ WEHI-3 cells. Specifically, BLAB/c mice were divided into 4 groups. Group I was injected i.p. with WEHI-3 only. Group II was compound 1 treatment (i.p. of 30 mg/kg/2 days for 14 days) started at 14 days after WEHI-3 cell injection. Group III was ATRA treatment (i.p. of 30 mg/kg/2 days for 14 days) started at 14 days after WEHI-3 cell injection. Group IV was compound 1 and ATRA treatment (i.p. of 30 mg/kg/2 days for 14 days) started at 14 days after WEHI-3 cell injection. Control mice and all groups of mice were treated for 2 weeks before the animals were weighed and the blood was draw and sacrificed for further experiments.

Results from these studies show that compound 1, ATRA, and compound 1 combined with ATRA significantly prolonged the mean survival time of WEHI-3/BALB/c mice (Table 1). The mean survival time of WEHI-3/BALB/c mice was 30 days, and this increased to 40, 42 and 44 days when WEHI-3/BALB/c mice were treated with 30 mg/kg/mouse of compound 1, ATRA, and compound 1 combined with ATRA, respectively (*P*<0.05, log-rank and generalized Wilcoxon's tests).

**Table 1. Effects of Compound 1, ATRA, and Compound 1 combined with ATRA on the survival of BALB/c mice bearing murine WEHI-3 leukemia cells.**

| Group/Treatment | No. of mice | Survival days | |
|---|---|---|---|
| | | Mean ± S.D. | Range |
| Group I Un-treated | 10 | 26.0 ±3.9 | 20-30 |
| Group II YC-1 (30 mg/kg/mouse; QD*7) | 10 | 32.3±4.9* | 22-40 |
| Group III ATRA (30 mg/kg/mouse; QD*7) | 10 | 33.9 ± 5.0* | 26-42 |
| Group IV YC-1 combined ATRA (30 mg/kg/ mouse; QD*7) | 10 | 35.9 ± 5.2** | 28-44 |

| | | | |
|---|---|---|---|
| QD*7: once two days by seven times **p* < 0.05, log-rank test; **p* < 0.05, generalized Wilcoxon's test. ***p* < 0.01, log-rank test; ***p* < 0.01, generalized Wilcoxon's test. | | | |

The spleen and liver tissues were isolated from individual animals, photographed, weighed, and histopathologically examined. Representative results are presented in Figs. 1 and 2. These data showed that compound 1, ATRA, and compound 1 combined with ATRA improved the body weight loss in leukemia mice within one month of experiment (data not shown). The enlargement of spleen, lymph nodes, liver metastases were significantly reduced in all treated groups, as compared with that in the untreated leukemia mice (FIG. 1). In addition, H-E stain of spleen section revealed that infiltration of immature myeloblastic cells into splenic red pulp was reduced in compound 1, ATRA, and compound 1 combined with ATRA treatment groups (FIG. 2).

To investigate the growth inhibition effects of compound 1, ATRA, and compound 1 combined with ATRA in WEHI-3 cells *in vitro,* cell cycle, NBT reduction with TUNEL staining were used to determine differentiation and apoptosis. After treating cells with compound 1 (5 µM), ATRA (1µM) and compound 1 combined with ATRA for 24 h, a pronounced G0/G1 arrest in the ATRA and compound 1 combined with ATRA-treated WEHI-3 cells was observed. This phenomenon did not occur in ATRA-treated WEHI-3 cells, but both compound 1 and compound 1 combined with ATRA-treated WEHI-3 cells have increased sub-G1 population (data not shown).

To explore the ability of compound 1 to induce apoptosis and ATRA to induce differentiation of WEHI-3 cells, NBT reduction activity and TUNEL/DAPI staining were assessed in WEHI-3 cells treated with compound 1, ATRA, and compound 1 combined with ATRA. After 24 h culture with compound 1 and compound 1 combined with ATRA, the cells exhibited nuclear shrinkage, chromatin condensation and DNA fragmentation. This phenomenon was not observed in ATRA-treated WEHI-3 cells (FIG. 3A). In contrast to the control cells, significant increases of NBT-reduction were observed in cells with ATRA and compound 1 combined with ATRA. The percentages of NBT positive cells were 3.56, 33.42 and 45.22 %, respectively, and TNUEL positive cells were 40.33, 5.45 and 45.22 %, respectively, after treatment with compound 1, ATRA, and compound 1 combined with ATRA (FIG. 3B). These results suggest that ATRA induced differentiation and compound 1 induced apoptosis in WEHI-3 cells.

For a further assessment of apoptosis, we examined the DNA fragmentation and caspase-3 activity assay in compound 1-treated WEHI-3 cells. Agarose gel electrophoresis showed that the DNA extracted from the WEHI-3 cells treated with compound 1 at 5 µM in 12 and 24 h were fragmented into a ladder of 180-200 base pairs (FIG. 4A). To determine whether the activation of caspase-3 is required for the induction of apoptosis by compound 1, we pretreated with or without caspase-3 inhibitor (Z-DEVD-FMK) in WEHI-3 cells for one hour before exposure to 50 µM of compound 1. As shown in FIG. 4B, 12, 18 and 24 h treatment of YC-1 caused a rapid induction of caspase-3 activity. The induced caspase-3 activity was blocked by Z-DEVD-FMK pretreatment. These results suggest that the induction of apoptosis by compound 1 is a specific biochemical event brought about by caspase-3 activity.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. For example, a compound structurally analogous to a fused pyrazolyl compound can also be used to practice the present invention. Thus, other embodiments are also within the claims.

## Claims

1. A composition for treating cancer, comprising a cancer chemotherapeutic agent and a compound of the formula: 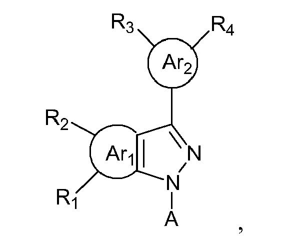 wherein
A is H or 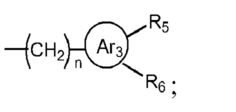 each of Ar₁, Ar₂, and Ar₃, independently, is phenyl, thienyl, furyl, pyrrolyl, pyridinyl, or pyrimidinyl;
each of R₁, R₂, R₃, R₄, R₅, and R₆, independently, is R, nitro, halogen, C(O)OR, C(O)SR, C(O)NRR', (CH₂)ₘOR, (CH₂)ₘSR, (CH₂)ₘNRR', (CH₂)ₘCN, (CH₂)ₘC(O)OR, (CH₂)ₘCHO, (CH₂)ₘCH=NOR, (CH₂)ₘC(O)N(OR)R', N(OR)R', or R₁ and R₂ together, R₃ and R₄ together, or R₅ and R₆ together are O(CH₂)ₘO, in which each of R and R', independently, is H or C₁~C₆ alkyl; and m is 0, 1,2, 3, 4, 5, or 6, and
n is 0, 1, 2, or 3.

2. The composition of claim 1, wherein A is

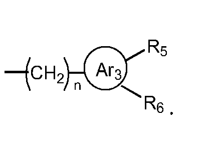

3. The composition of any of claims 1-2, wherein Ar₃ is phenyl.

4. The composition of claim 1, wherein A is H.

5. The composition of any of claims 1-4, wherein Ar₁ is phenyl.

6. The composition of any of claims 1-5, wherein Ar₂ is furyl.

7. The composition of any of claims 1-6, wherein one of R₃ and R₄ is H, and the other is CH₂OH

8. The composition of any of claims 1-7, wherein each of R₁, R₂, R₅, and R₆ is H.

9. The composition of any of claims 1-8, wherein the chemotherapeutic agent is a DNA-binding chemotherapeutic agent.

10. The composition of any of claims 1-8, wherein the chemotherapeutic agent is a microtubule-stabilizing agent.
